(19) Europäisches Patentamt
European Patent Office
Office européen des brevets

(11) Veröffentlichungsnummer : **0 282 531 B1**

# (12) EUROPÄISCHE PATENTSCHRIFT

(45) Veröffentlichungstag der Patentschrift : **15.04.92 Patentblatt 92/16**

(51) Int. Cl.$^5$ : **C07D 501/46**

(21) Anmeldenummer : **87905949.1**

(22) Anmeldetag : **09.09.87**

(86) Internationale Anmeldenummer : **PCT/EP87/00510**

(87) Internationale Veröffentlichungsnummer : **WO 88/02001 24.03.88 Gazette 88/07**

## (54) STABILE, KRISTALLINE FORM EINES CEFALOSPORIN-ZWISCHENPRODUKTES.

Die Akte enthält technische Angaben, die nach dem Eingang der Anmeldung eingereicht wurden und die nicht in dieser Patentschrift enthalten sind.

(30) Priorität : **10.09.86 AT 2428/86**
**10.09.86 AT 2427/86**

(43) Veröffentlichungstag der Anmeldung : **21.09.88 Patentblatt 88/38**

(45) Bekanntmachung des Hinweises auf die Patenterteilung : **15.04.92 Patentblatt 92/16**

(84) Benannte Vertragsstaaten : **AT BE CH DE FR GB IT LI LU NL SE**

(56) Entgegenhaltungen :
**EP-A- 0 166 580**
**WO-A-85/04659**
**US-A- 4 258 041**

(73) Patentinhaber : **BIOCHEMIE GESELLSCHAFT M.B.H.**
**A-6250 Kundl (AT)**

(72) Erfinder : **PRAGER, Bernhard Christian**
**Steinbacherstrasse 1**
**A-6300 Wörgl (AT)**
Erfinder : **WESSELY, Karl**
**Schützenstrasse 12**
**A-6330 Kufstein (AT)**
Erfinder : **VEIT, Werner**
**Kaiserjägerstrasse 27**
**A-6330 Kufstein (AT)**

(74) Vertreter : **Grange, Gordon et al**
**c/o SANDOZ AG Patentabteilung**
**CH-4002 Basel (CH)**

**EP 0 282 531 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine neue stabile, kristalline Form des (6R,7R)-7-[[2-(2-Amino-4-thiazolyl)-(Z)-2-(1-tert.butoxycarbonyl-1-methylethoxy)-imino]acetamido]-3-(1-pyridiniummethyl)-3-cephem-4-carboxylats (Ceftazidime-t-butylester) der Formel

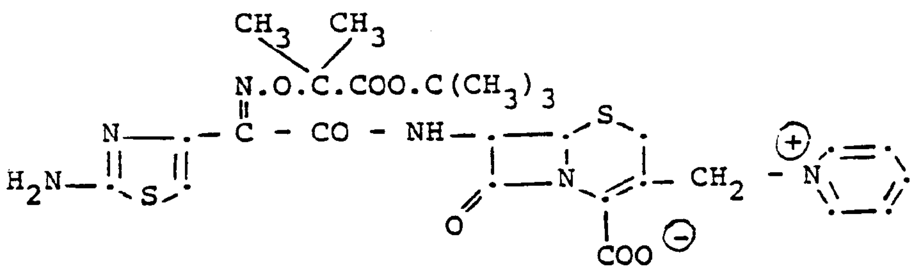

I

und Verfahren zu dessen Herstellung.

Die Verbindung der Formel I ist ein wichtiges Zwischenprodukt zur Herstellung von Ceftazidime. Ceftazidime ist ein hochaktives Antibiotikum, welches durch seine breitgefächerte Wirkung gegen Mikroorganismen, speziell im gramnegativen Bereich, außerordentliche Bedeutung erlangt hat und als parentereles Pharmazeutikum, vor allem im Hospitalbereich, benützt wird, wobei euch seine Wirkung gegen Problemkeime, wie Pseudomonas, von Bedeutung ist.

Die Herstellung von Ceftezidime erfolgt üblichewreise über Zwischenstufen, die Schutzgruppen an der Aminogruppe des Thiazolrings, an der Carboxylgruppe in der Seitenkette und manchmal auch an der Carboxylgruppe des Thiazolidinrings enthalten. Diese Schutzgruppen werden in einem oder mehreren Reaktionsschritten abgespalten.

Die Verbindung der Formel I zeichnet sich unter diesen geschützten Ceftazidimezwischenstufen besonders aus, da bei ihrer Weiterverarbeitung zu Ceftazidime, z.B. durch saure Hydrolyse, als Nebenprodukt nur Isobuten entsteht und das Ceftazidime in hoher Reinheit und guter Ausbeute isoliert werden kann. In der W0 85/4659 wird eine besonders einfache Methode zur Herstellung der Verbindung der Formel I durch Umsetzung des aktivierten Thiolesters der Formel

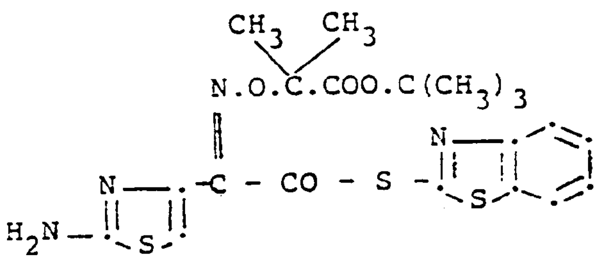

II

in der syn-Form mit der Verbindung der Formel

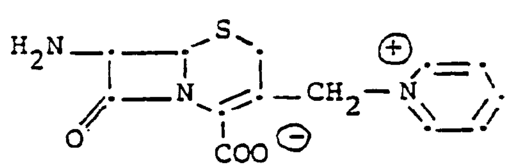

IIIa

beschrieben. Nach dem in dieser Anmeldung angegebenen Verfahren wird die Verbindung der Formel I jedoch nur in amorpher, schwer isolierbarer Form erhalten.

Es wurde nun gefunden, daß daß bei Verwendung geeigneter Lösungsmittel die Verbindung der Formel I durch einfache Filtration aus dem Reaktionsgemisch in kristalliner Form und in guter Ausbeute und Reinheit isoliert werden kann, wobei die Nebenprodukte und Verunreinigungen in Lösung bleiben.

Geeignete Lösungsmittel sind halogenierte aliphatische Kohlenwasserstoffe, z.B. Dichlormethan oder Chloroform, niedere aliphatische Alkohole, z.B. Methanol bis n-Butenol, niedere aliphatische Carbonsäureester, z.B. Ethylacetat oder Butylacetat, Dimethylformamid oder Dimethylsulfoxid und Gemische aus diesen

Lösungsmitteln. Auch Gemische aus niederen Alkoholen, Dimethylformamid oder Dimethylsulfoxid einerseits und aromatischen Kohlenwasserstoffen, z.B. Benzol, Toluol, Chlorbenzole, oder Aceton oder Acetonitril andererseits, können als Lösungsmittel verwendet werden. Bevorzugte Lösungsmittel sind Mischungen aus Dichlormethan, Chloroform, Butylacetat, Ethylacetat oder Toluol einerseits und Methanol oder Dimethylsulfoxid andererseits. Die Mischverhältnisse bewegen sich in der Regel zwischen 20:1 und 1:20 und richten sich nach der Löslichkeit des Endproduktes.

Der aktivierte Thiolester der Formel II wird in bekannter Weise, z.B. durch Umsetzung der entsprechenden Säure mit Bis-(benzothiazol-2-yl)disulfid und Triphenylphosphin hergestellt, wobei das Reaktionsgemisch ohne Isolierung des Thiolesters für die Umsetzung mit der Verbindung der Formel IIIa eingesetzt werden kann. Die Verwendung von isoliertem Thiolester gibt jedoch bessere Ausbeuten und reinere Produkte.

Die Verbindung der Formel IIIa kann als inneres Salz oder als Additionssalz einer organischen oder anorganischen Säure eingesetzt werden. Auch Solvate dieser Verbindungen können verwendet werden. Beispiele für verwendete Formen der Verbindung der Formel IIIa sind das Dihydrat, Monohydrochlorid.Monohydrat, Dihydrochlorid.Dihydrat, Hydrojodid.Monohydrat und Oxalat. Bei Verwendung von Säureadditionssalzen muß spätestens vor der Produktfiltration eine der Säure äquivalente Menge an Base (z.B. Triethylamin) zugegeben werden.

Die Verbindung der Formel IIIa kann daher allgemein folgendermaßen definiert werden:

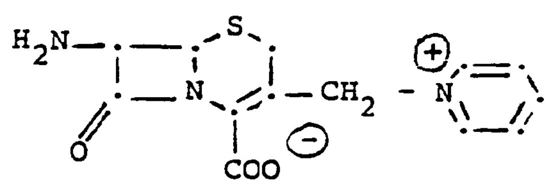 $\cdot\ (HX)_n \cdot (LM)_m$ III

wobei HX eine organische oder anorganische Säure, LM Wasser oder ein organisches Lösungsmittel bedeuten und n und m für 0, 1 oder 2 stehen.

Die Reaktionstemperaturen liegen vorzugsweise zwischen 0° C und Raumtemperatur. Die Reaktion kann jedoch auch bei tieferen oder höheren Temperaturen durchgeführt werden, wobei aber mit längeren Reaktionszeiten bzw. verstärkten Nebenreaktionen gerechnet werden muß.

Die Filtration des Produktes wird normalerweise bei der gleichen Temperatur wie die Reaktion durchgeführt; zur Erhöhung der Ausbeute oder zur Verbesserung der Produktreinheit kann die Filtration auch bei tieferer bzw. höherer Temperatur erfolgen. Aus den gleichen Gründen (Ausbeute, Reinheit) kann das Reaktionsgemisch vor der Filtration noch mit einem bereits verwendeten oder einem zusätzlichen Lösungsmittel verdünnt oder ein Teil des eingesetzten Lösungsmittels abdestilliert werden.

Wie schon erwähnt, wird so der Ceftazidime-t-butylester kristallin in Form von kleinen, unter dem Polyrisationsmikroskop doppelbrechenden Nadeln erhalten. Die Kristallinität des Produktes wurde durch Röntgenbeugungsmessung an einer Pulverprobe bestätigt:

| d (Å) | I |
|---|---|
| 17,0 | w |
| 15,5 | m |
| 5,9 | w |
| 5,0 | w |
| 4,15 | w |
| 3,84 | w |
| 3,3 | w |

w = schwach
m = mittel

Diese kristalline Form wird in der Folge als α-Form bezeichnet. Diese α -Form wird nach dem erfindungsgemäßen Verfahren in hoher Ausbeute erhalten, besitzt jedoch eine relativ geringe Lagerstabilität. Lagert man z.B. dieses Produkt einige Tage bei Raumtemperatur (~ 25° C), so ist eine Zersetzung zu beobachten. Diese Zersetzlichkeit (von Geruch nach Pyridin begleitet) ist unerwünscht und führt neben Problemen bei der Trocknung auch zu Problemen und erhöhtem Aufwand bei der Lagerung und Weiterverarbeitung.

Es wurde nun weiters gefunden, daß die Verbindung der Formel I auch in einer anderen, stabileren Form und höchster Reinheit (≥ 97 %) erhalten werden kann. Diese neue kristalline Modifikation, im folgenden β-Form genannt, kann aus der amorphen Form oder auch durch Modifikation der Isolierung des Verfahrens zur Herstellung der α-Form, ohne oder mit Zwischenisolierung der α-Form, hergestellt werden. Die neue Modifikation liegt als kristallines Pulver vor, welches unter dem Polarisationsmikroskop als kleine doppelbrechende Nadel erscheint und dessen Röntgenspektrum sich charakteristisch von dem der α-Form unterscheidet:

| d (Å) | I |
|---|---|
| 22,0 | m |
| 10,0 | w |
| 8,5 | m |
| 7,3 | w |
| 6,7 | m |
| 5,9 | md |
| 5,6 | m |
| 5,3 | wd |
| 4,2 | w |
| 3,94 | w |
| 3,82 | w |
| 3,7 | w |
| 3,3 | w |

w: schwach
m: mittel
d: diffus

Diese neue β-Form der Verbindung der Formel I wird dem vorliegenden erfindungsgemäßen Verfahren entsprechend aus wäßriger Phase gewonnen, wobei die wäßrige Phase auch mit Wasser mischbare Lösungsmittel enthalten kann.

Das erfindungsgemäße Verfahren ist dadurch gekennzeichnet, daß man die im Reaktionsgemisch in amorpher oder in α-Form enthaltene Verbindung der Formel I durch Extraktion mit Wasser oder mit einem Gemisch von Wasser und einem mit Wasser mischbaren Lösungsmittel, wobei mindestens 50 % Wasser verwendet werden, bei einem pH-Wert unter 3, insbesondere bei pH 1,0 bis 2,0, in eine wäßrige Phase überführt, die auch mit Wasser mischbare Lösungsmittel enthalten kann, und aus dieser durch Einstellen des pH-Wertes in den Bereich des isoelektrischen Punktes der Verbindung der Formel I oder darüber, also auf den Bereich von 3,5 bis 7,5, insbesondere auf 4,2 bis 5,2, die Verbindung der Formel I in β-Form zum Kristallisieren bringt. Die Verbindung der Formel I wird dabei in Form eines hochkristallinen, farblosen Niederschlages erhalten, welcher leicht durch Filtration isolierbar ist. Man kann jedoch auch die isolierte Verbindung in amorpher oder in α-Form in Wasser oder in einem Gemisch aus Wasser und einem oder mehreren mit Wasser mischbaren Lösungsmitteln - vorteilhafterweise in hochkonzentrierter Lösung - eventuell unter Zusatz einer Säure bis etwa pH 2,0 oder darunter, lösen, dann die erfindungsgemäße neue, kristalline β-Form zum Auskristallisieren bringen, indem man den pH-Wert auf 3,5 - 7,5, insbesondere auf 4,2 - 5,2 einstellt, und erhält dadurch einen farblosen kristallinen Niederschlag, der sich leicht isolieren läßt und die erfindungsgemäße β-Form der Verbindung der Formel I in hoher Reinheit ( ≥ 98 %) darstellt.

Geht man, wie beschrieben, von der amorphen bzw. der isolierten α-Form aus, so ist das Einstellen des pH-Wertes auf unter 3,0 nicht unbedingt nötig, da sich die α-Form auch im Neutralbereich bzw. bei pH-Werten zwischen 3,5 und 7,5 gut in Wasser oder Gemischen von Wasser und mit Wasser mischbaren Lösungsmitteln löst, doch kann es beim Lösen vorkommen, daß die neue erfindungsgemäße β-Form der Verbindung der Formel I bereits auszurukristallisieren beginnt, bevor noch alle α-Form in Lösung gegangen ist. Dies tritt speziell beim Arbeiten in höher konzentrierten Lösungen ( > 10 %) bei Verwendung von reinem Wasser ohne Lösungsmittelzusatz auf. Prinzipiell ist es nicht nötig, daß alle α-Form in Lösung gebracht wurde, bevor die neue erfindungsgemäße β-Form ausfällt, da auch beim Nichterreichen einer zwischenzeitlich klaren Lösung sich in der vorhandenen Suspension (bereits ausgefallene neue β-Form vorhanden) das Startmaterial völlig löst und in die neue erfindungsgemäße β-Form umwandelt.

Zwecks besserer Übersichtlichkeit, speziell in größerem Maßstab, und um eine stabile klare Lösung (für eventuelle Filtration von Fremdkörpern oder noch Zusatz von Hilfsstoffen wie Aktivkohle etc. zu Reinigungszwecken) zu erhalten, ist jedoch die Herstellung einer klaren beständigen Lösung (ohne das Auskristallisieren der neuen β-form erwarten zu müssen) erstrebenswert. Dies kann durch Zusatz einer Säure erreicht werden, wobei günstigerweise mindestens 1 Äquivalent Säure verwendet wird, wodurch die Verbindung der Formel I als Säure-Addukt (Salz) in Lösung gehalten wird. Zum Gewinn der gewünschten erfindungsgemäßen neuen

kristallinen β-Form setzt man dann jene Menge an Base zu, die die vorher eingebrachte Säure neutralisiert, wobei dann die neue β-Form der Verbindung der Formel I aus der wäßrigen Lösung auskristallisiert und isoliert werden kann.

Als Säure - sowohl beim Isolieren aus dem Reaktionsgemisch, wie auch für die Herstellung der neuen β-Form durch Umwandlung aus der isolierten α-Form können alle herkömmlichen anorganischen und organischen Säuren verwendet werden, vor allem Schwefelsäure und Salzsäure, insbesondere geeignet ist Salzsäure.

Als mit Wasser mischbare Lösungsmittel können Alkohole, Aceton, Acetonitril und solche eingesetzt werden, in denen sich die erfindungsgemäße β-Form der Verbindung der Formel I nicht oder nur schlecht löst. Von den Alkoholen sind deshalb Ethanol, Isopropanol, n-Propanol und auch n-Butanol geeignet, während Methanol nur in geringer Konzentration geeignet ist, da es in höherer Konzentration die Löslichkeit der neuen β-Form der Verbindung der Formel I zu sehr erhöht. Im allgemeinen ist der Zusatz von mit Wasser mischbaren Lösungsmitteln nicht nötig, und günstigerweise wird nur Wasser als Extraktions- oder Umfällmedium verwendet, da die Löslichkeit der neuen erfindungsgemäßen β-Form der Verbindung der Formel I dabei am geringsten ist und dadurch die höchsten Ausbeuten erzielt werden.

In den folgenden Beispielen, die die Erfindung näher erläutern, ihren Umfang jedoch in keiner Weise einschränken sollen, erfolgen alle Temperaturangaben in Celsiusgraden. Die spezifische optische Drehung $[\alpha]_D^{20}$ wurde in Wasser/Methanol 1:1 bei einer Konzentration von 1 g/100 ml gemessen.

Die Identität der Produkte wurde durch NMR überprüft: $^1$H-NMR (90 MHz, DMSO-$d_6$/$D_2O$/DCL): 8,1 bis 9,1 (5H, Pyridinium); 7,15 (1H, s, Thiazol); 5,92 (1H, d, J = 5 Hz, $H_7$); 5,6 (2H, $CH_2$-N); 5,32 (1H, d, J = 5Hz, $H_6$); 3,6 (2H, S-$CH_2$); 1,55 (6H, s, $C(CH_3)_2$); 1,40 (9H, $C(CH_3)_3$).

Beispiel 1: (6R,7R)-7-[[2-(2-Amino-4-thiazolyl)-(Z)-2-(1-tert. butoxycarbonyl-1-methylethoxy)imino]acetamido]-3-(1-pyridiniummethyl)-3-cephem-4-carboxylat (α-Form):

12 g 2-(2-Amino-4-thiazolyl)-(Z)-2[(1-tert.butoxycarbonyl-1-methylethoxy)imino]thioessigsäure-S-benzothiazol-2-ylester, 7,7 g (6R,7R)-7-Amino-3-(1-pyridiniummethyl)-3-cephem-4-carbonsäure.Chlorid.Monohydrat und 3,8 ml Triethylamin werden in einem Gemisch aus 75 ml Dichlormethan und 5 ml Methanol 10 Stunden bei 0° gerührt. Der Feststoff wird abfiltriert, mit Dichlormethan gewaschen und bei Raumtemperatur im Vakuum getrocknet. Man erhält 10,5 g der Titelverbindung in Form eines farblosen Kristallpulvers (= 78 % der Theorie). Reinheit: 97 %; $[\alpha]_D^{20}$ = -36,4°.

Beispiel 2: (6R,7R)-7-[[2-(2-Amino-4-thiazolyl)-(Z)-2-(1-tert.butoxycarbonyl-1-methylethoxy)imino]acetamido]-3-(1-pyridiniummethyl)-3-cephem-4-carboxylat (α-Form):

Ein Ansatz nach Beispiel 1 wird in 80 ml Dichlormethan 4 Stunden bei Raumtemperatur gerührt. Die Reaktionssuspension wird auf 40° erwärmt und der Feststoff abfiltriert. Man erhält 10 g der Titelverbindung (= 75 % der Theorie) in kristalliner Form. Reinheit: 95 %; $[\alpha]_D{}^{20}$ = -36,2°.

Beispiel 3: (6R,7R)-7-[[2-(2-Amino-4-thiazolyl)-(Z)-2-(1-tert.butoxycarbonyl-1-methylethoxy)imino]acetamido]-3-(1-pyridiniummethyl)-3-cephem-4-carboxylat (α-Form):

Ein Ansatz nach Beispiel 1 wird in 50 ml Methanol 20 Stunden bei 0° und dann 2 Stunden bei -20° gerührt. Der Feststoff wird wie in Beispiel 1 isoliert. Man erhält 9 g der Titelverbindung (= 67 % der Theorie) in kristalliner Form. Reinheit: 98 %; $[\alpha]_D^{20}$ = -35,6°..

Beispiel 4: (6R,7R)-7-[[2-(2-Amino-4-thiazolyl)-(Z)-2-(1-tert.butoxycarbonyl-1-methylethoxy)imino]acetamido]-3-(1-pyridiniummethyl)-3-cephem-4-carboxylat (α-Form):

Die Reaktion wird wie in Beispiel 1, jedoch in einem Gemisch aus 70 ml Dichlormethan und 10 ml Dimethylsulfoxid, durchgeführt. Man erhält 8 g der Titelverbindung (= 60 % der Theorie) als Kristalle. Reinheit: 95 %; $[\alpha]_D^{20}$ = -35,8 °,

Beispiel 5: (6R,7R)-7-[[2-(2-Amino-4-thiazolyl)-(Z)-2-(1-tert.butoxycarbonyl-1-methylethoxy)imino]acetamido]-3-(1-pyridiniummethyl)-3-cephem-4-carboxylat (α-Form):

2,4 g 2-(2-Amino-4-thiazolyl)-(Z)-2-[(1-tert.butoxycarbonyl-1-methylethoxy)imino]thioessigsäure-S-benzothiazol-2-ylester und 1,3 g (6R,7R)-7-Amino-3-(1-pyridiniummethyl)-3-cephem-4-carboxylat.Monohydrat werden in einem Gemisch aus 15 ml Dichlormethan und 5 ml Methanol 6 Stunden bei 0° gerührt. Der Feststoft wird wie in Beispiell isoliert. Man erhält 1,9 g der Titelverbindung (= 75 % der Theorie) in kristalliner Form. Reinheit: 98 %; $[\alpha]_D^{20}$ = -36,5°.

Beispiel 6: (6R,7R)-7-[[2-(2-Amino-4-thiazolyl)-(Z)-2-(tert.butoxycarbonyl-1-methylethoxy)imino]acetamido]-3-(1-pyridiniummethyl)-3-cephem-4-carboxylat (α-Form):

19,1 g 2-(2-Amino-4-thiazolyl)-(Z)-2[(1-tert.butoxycarbonyl-1-methylethoxy)imino]thioessigsäure-S-benzothiazol-2-ylester, 14,6 g (6R,7R)-7-Amino-3-(1-pyridiniummethyl)-3-cephem-4-carbonsäure.Jodid.Monohydrat und 5,6 ml Triethylamin werden in einem Gemisch aus 142,5 ml Dichlormethan und 7,5 ml Methanol 24 Stunden bei 0° gerührt. Der Feststoff wird abfiltriert, mit Dichlormethan gewaschen und bei Raumtemperatur im Vakuum getrocknet. Man erhält 16,0 g der Titelverbindung (= 79,6 % der Theorie) in kristalliner Form. Reinheit: 97 %; $[\alpha]_D^{20}$ = -36,7°.

Beispiel 7: (6R,7R)-7-[[2-Amino--4-thiazolyl)-(Z)-2-(1-tert.butoxycarbonyl-1-methylethoxy)imino]acetamido]-3-(1-pyridiniummethyl)-3-cephem-4-carboxylat (β-Form):

40 g (6R,7R)-7-[[2-(2-Amino-4-thiezolyl)-(Z)-2-(1-tert.butoxycarbonyl-1-methylethoxy)imino]acetamido]-3-(1-pyridiniummethyl)-3-cephem-4-carboxylat (α-Form) werden in 400 ml Wasser gelöst. Man beimpft mit Kristallen der β-Form, wodurch sich ein farbloser kristalliner Niederschlag bildet. Nach Kühlung auf etwa + 4° werden die Kristalle isoliert und man erhält nach Waschen mit Wasser und Trocknung 27 g der β-Form.

Beispiel 8: (6R,7R)-7-[[2-(2-Amino-4-thiazolyl)-(Z)-2-(1-tert.butoxycarbonyl-1-methylethoxy)imino]acetamido]-3-(1-pyridiniummethyl)-3-cephem-4-carboxylat (β-Form):

24 g 2-(2-Amino-4-thiazolyl)-(Z)-2[(1-tert.butoxycarbonyl-1-methylethoxy)imino]thioessigsäure-S-benzothiazol-2-yl-ester, 15,4 g (6R,7R)-7- Amino-3-(1-pyridiniummethyl)-3-cephem-4-carbonsäure.Chlorid und 7,6 ml Triethylamin werden in einem Gemisch aus 150 ml Dichlormethan und 5 ml Methanol bei 0° 20 Stunden gerührt, Das Reaktionsgemisch wird dann mit 100 ml Wasser und 20 ml HCl konz. versetzt und die Phasen getrennt. Die kalte wäßrige Phase wird filtriert und dann der pH-Wert der Lösung mit 3 N NaOH auf 4,8 - 5,0 gestellt, wobei die Temperatur auf 20 - 22° ansteigt und die Titelverbindung auskristallisiert. Nach Abkühlung auf etwa 4° für 2 Stunden filtriert man die Kristalle ab, wäscht mit wenig kaltem Wasser und erhält nach Trocknung im Vakuum 16 g der Titelverbindung in der β-Form.

Beispiel 9: (6R,7R)-7-[[2-(2-Amino-4-thiazolyl)-(Z)-2-(1-tert.butoxycarbonyl-1-methylethoxy)imino]acetamido]-3-(1-pyridiniummethyl)-3-cephem-4-carboxylat (β-Form):

70 g (6R,7R)-7-[[(2-(2-Amino-4-thiezolyl)-(Z)-2-(1-tert.butoxycarbonyl-1-methylethoxy)imino]acetamido]-3-(1-pyridiniummethyl)-3-cephem-4-carboxylat (α-Form; 85 %ig) werden in eine Mischung von 25 ml HCl konz. und 500 ml Eiswasser eingetragen. Man erhält eine klare Lösung von etwa pH 1,5. Es wird nun mit 2 N NaOH der pH-Wert auf 4,8 - 5,0 eingestellt und dabei die Temperatur auf 18 - 22° erhöht. Dabei kristallisiert die Titelverbindung in Form eines farblosen Niederschlages aus. Es wird anschließend 2 Stunden auf +4° gekühlt und dann die Kristalle isoliert, mit kaltem Wasser (100 ml) gewaschen und der wasserfeuchte Kuchen in der Wirbelschicht mit Luft von 45 - 50° getrocknet. Man erhält auf diese Weise 52 g hochreiner ($\geq$ 98 %) Titelverbindung der β-Form mit einem $H_2O$-Gehalt von 8,6 %.

## Patentansprüche

1. Neue kristalline, stabile Form des (6R,7R)-7-[[2-(2-Amino-4-thiazolyl)-(Z)-2-(1-tert.butoxycarbonyl-1-methylethoxy)imino]acetamido]-3-(1-pyridiniummethyl)-3-cephem-4-carboxylats (Ceftazidime-t-butylester) der Formel

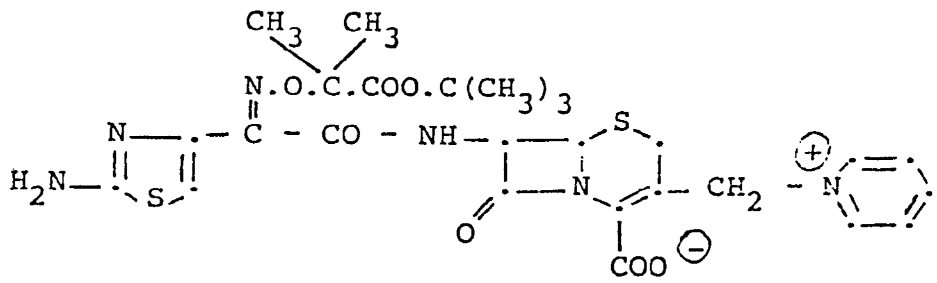

I

mit dem Röntgenspektrum

| d (Å) | I |
| --- | --- |
| 17,0 | w |
| 15,5 | m |
| 5,9 | w |
| 5,0 | w |
| 4,15 | w |
| 3,84 | w |
| 3,3 | w |

w = schwach
m = mittel

(α-Form).

2. Verfahren zur Herstellung einer neuen kristallinen, stabilen Form des (6R,7R)-7-[[2-(2-Amino-4-thiazolyl)-(Z)-2(1-tert.butoxycarbonyl-1-methylethoxy)imino]acetamido]-3-(1-pyridiniummethyl)-3-cephem-4-carbo xylats (α-Form) nach Anspruch 1 durch Umsetzung der Verbindung der Formel

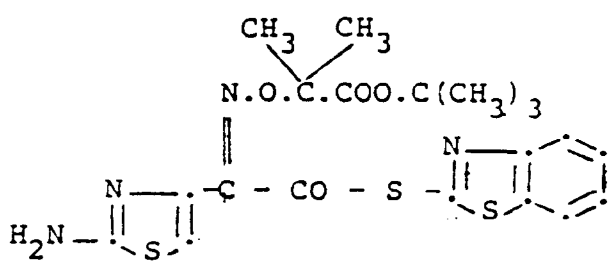

II

in der syn-Form mit einer Verbindung der Formel

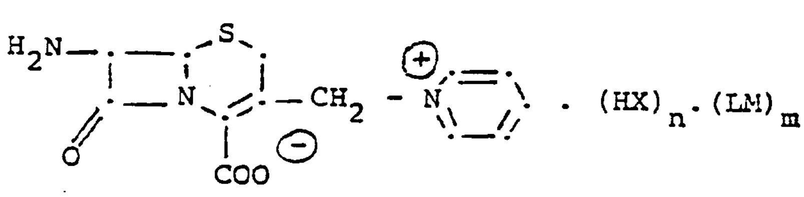

III

wobei HX eine anorganische oder organische Säure bedeutet, LM für Wasser oder ein organisches Lösungsmittel steht und n und m für 0, 1 oder 2 stehen, gegebenenfalls unter Zusatz einer Base, dadurch gekennzeichnet, daß man die Reaktion in einem Lösungsmittel oder Lösungsmittelgemisch durchführt, in dem die Verbindung der Formel I schwer löslich ist, beispielsweise halogenierte Kohlenwasserstoffe, niedere aliphatische Carbonsäureester, niedere aliphatische Alkohole, Dimethylformamid oder Dimethylsulfoxid oder Gemische dieser Lösungsmittel, und man die Verbindung der Formel I unmittelbar nach erfolgter Umsetzung aus dem Reaktionsgemisch abfiltriert.

3. Verfahren nach Anspruch 2, dadurch gekennzeichnet, daß man als Lösungsmittel Gemische aus niederen aliphatischen Alkoholen, Dimethylformamid oder Dimethylsulfoxid einerseits und aromatischen Kohlenwasserstoffen, Aceton oder Acetonitril andererseits verwendet.

4. Verfahren nach Anspruch 2, dadurch gekennzeichnet, daß man Gemische aus Dichlormethan und Methanol verwendet.

5. Neue kristalline, stabile Form des (6R,7R)-7-[[2-(2-Amino-4-thiazolyl)-(Z)-2-(1-tert.butoxycarbonyl-1-methylethoxy)imino]acetamido]-3-(1-pyridiniummethyl)-3-cephem-4-carboxylats (Ceftazidime-t-butylester) der Formel I wie in Anspruch 1 definiert mit dem Röntgenspektrum

| d (Å) | I | |
|---|---|---|
| 22,0 | m | |
| 10,0 | w | |
| 8,5 | m | |
| 7,3 | w | |
| 6,7 | m | |
| 5,9 | md | w: schwach |
| 5,6 | m | m: mittel |
| 5,3 | wd | d: diffus |
| 4,2 | w | |
| 3,94 | w | |
| 3,82 | w | |
| 3,7 | w | |
| 3,3 | w | |

(β-Form).

6. Verfahren zur Herstellung der neuen kristallinen, stabilen Form der Verbindung der Formel I (β-Form) nach Anspruch 5, dadurch gekennzeichnet, daß man die Verbindung der Formel I aus wäßriger Lösung bei pH 3,5 -7,5 kristallisiert.

7. Verbindung der Formel I nach Anspruch 1 oder 5 zur Verwendung als Zwischenprodukt für die Herstellung von Ceftazidime.

## Claims

1. New crystalline, stable form of (6R,7R)-7-[[2-(2-amino-4-thiazolyl)-(Z)-2-(1-tert.butoxycarbonyl-1-methylethoxy)imino]acetamido]-3-(1-pyridiniummethyl)-3-cephem-4-carboxylate (ceftazidime-t-butylester) of formula

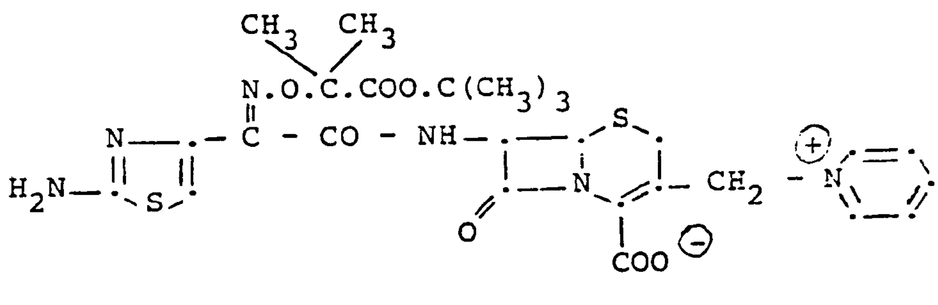

I

having the X-ray spectrum

| d (Å) | I |
|---|---|
| 17.0 | w |
| 15.5 | m |
| 5.9 | w |
| 5.0 | w |
| 4.15 | w |
| 3.84 | w |
| 3.3 | w |

w = weak
m = medium

(α-form).

2. Process for the production of a new crystalline, stable form of (6R,7R)-7-[[2-(2-amino-4-thiazolyl)-(Z)-2-(1-tert.butoxycarbonyl-1-methylethoxy)imino]acetamido]-3-(1-pyridiniummethyl)-3-cephem-4-carboxylate (α-form) according to claim 1, by reacting the compound of formula

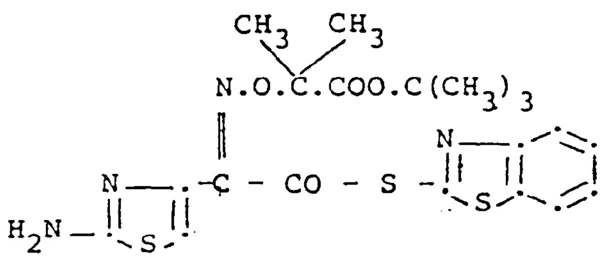

II

in syn-form with a compound of formula

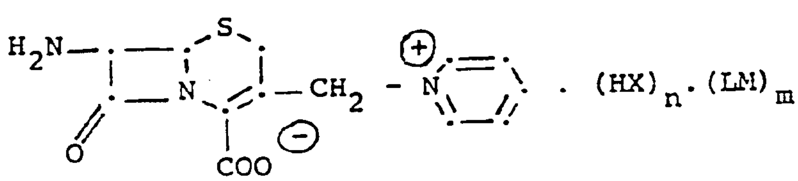

III

whereby HX signifies an inorganic or organic acid, LM denotes water or an organic solvent, and n and m are 0, 1 or 2, optionally adding a base, characterised in that the reaction is carried out in a solvent or solvent mixture in which the compound of formula I is poorly soluble, for example halogenated hydrocarbons, lower aliphatic carboxylic acid esters, lower aliphatic alcohols, dimethylformamide or dimethyl sulfoxide or mixtures of these solvents, and the compound of formula I is filtered off from the reaction mixture directly after the reaction.

3. Process according to claim 2, characterised in that as solvents mixtures of lower aliphatic alcohols, dimethylformamide or dimethyl sulphoxide on the one hand and aromatic hydrocarbons, acetone or acetonitrile on the other hand are used.

4. Process according to claim 2, characterised in that mixtures of dichloromethane and methanol are used.

5. New crystalline, stable form of (6R,7R)-7-[[2-(2-amino-4-thiazolyl)-(Z)-2-(1-tert.butoxycarbonyl-1-methylethoxy)imino]-acetamido]-3-(1-pyridiniummethyl)-3-cephem-4-carboxylate (ceftazidime-t-butylester) of formula I, as defined in claim 1, having the X-ray spectrum

| d (Å) | I |
|---|---|
| 22.0 | m |
| 10.0 | w |
| 8.5 | m |
| 7.3 | w |
| 6.7 | m |
| 5.9 | md |
| 5.6 | m |
| 5.3 | wd |
| 4.2 | w |
| 3.94 | w |
| 3.82 | w |
| 3.7 | w |
| 3.3 | w |

w = weak
m = medium
d = diffuse

(β-form).

6. Process for the production of the new crystalline, stable form of the compound of formula I (β-form) according to claim 5, characterised in that the compound of formula I is crystallised from an aqueous solution at pH 3.5 - 7.5.

7. Compound of formula I according to claim 1 or 5 for use as intermediate product for the production of ceftazidime.

## Revendications

1. Nouvelle forme cristalline stable du (6R,7R)-7-[[2-(2-amino-4-thiazolyl)-(Z)-2-(1-tert.-butoxycarbonyl-1-méthyléthoxy)imino]acétamido]-3-(1-pyridiniométhyl)-3-céphème-4-carboxylate (ester tert.-butylique de la ceftazidime) de formule

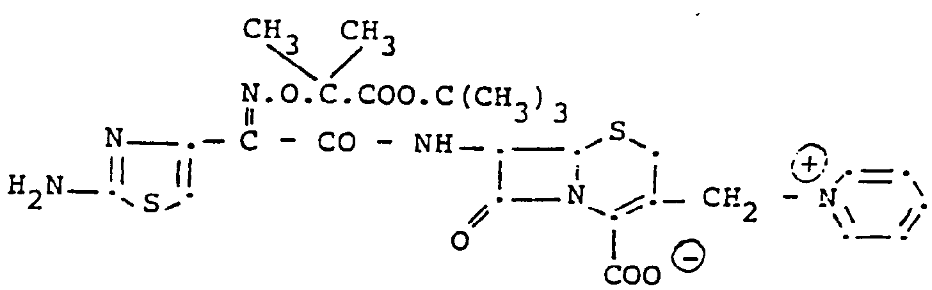

I

ayant le spectre aux rayons X suivant

| d(Å) | I |
|---|---|
| 17,0 | w |
| 15,5 | m |
| 5,9 | w |
| 5,0 | w |
| 4,15 | w |
| 3,84 | w |
| 3,3 | w |

w = faible
m = moyen

(forme α).

2. Procédé de préparation d'une nouvelle forme cristalline stable du (6R,7R)-7-[[2-(2-amino-4-thiazolyl)-(Z)-2-(1-tert.-butoxycarbonyl-1-méthyléthoxy)imino]acétamido]-3-(1-pyridiniométhyl)-3-céphème-4-carboxylate (forme α) selon la revendication 1, par réaction du composé de formule

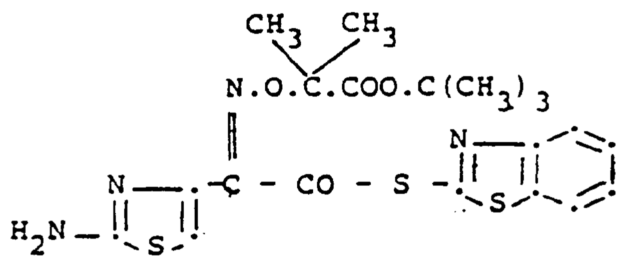

II

sous forme syn, avec un composé de formule

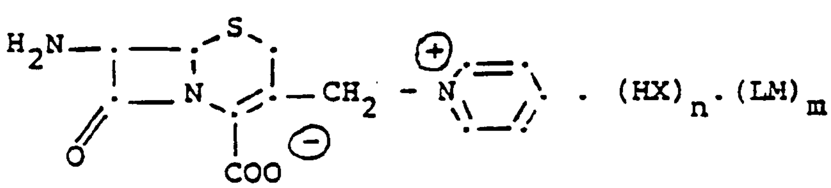

III

où HX signifie un acide minéral ou organique, LM signifie l'eau ou un solvant organique et n et m signifient 0, 1 ou 2, éventuellement sous addition d'une base, caractérisé en ce que l'on effectue la réaction dans un solvant ou dans un mélange de solvants, dans lequel le composé de formule I est difficilement soluble, par exemple un hydrocarbure halogéné, un ester d'acide carboxylique aliphatique inférieur, un alcool aliphatique inférieur, le diéthylformamide ou le diméthylsulfoxyde, ou un mélange de ces solvants, et on sépare par filtration, directement après la réaction, le composé de formule I du mélange réactionnel.

3. Procédé selon la revendication 2, caractérisé en ce qu'on utilise comme solvant un mélange d'une part d'un alcool aliphatique inférieur, de diméthylformamide ou de diméthylsulfoxyde, et d'autre part d'un hydrocarbure aromatique, d'acétone ou d'acétonitrile.

4. Procédé selon la revendication 2, caractérisé en ce qu'on utilise un mélange de dichlorométhane et de méthanol.

5. Nouvelle forme cristalline stable du (6R,7R)-7-[[2-(2-amino-4-thiazolyl)-(Z)-2-(1-tert.-butoxycarbonyl-1-méthyléthoxy)imino]acétamido]-3-(1-pyridiniométhyl)-3-céphème-4-carboxylate (ester tert.-butylique de la ceftazidime) de formule I telle que définie à la revendication 1, ayant le spectre aux rayons X suivant:

| d (Å) | I |
|---|---|
| 22,0 | m |
| 10,0 | w |
| 8,5 | m |
| 7,3 | w |
| 6,7 | m |
| 5,9 | md |
| 5,6 | m |
| 5,3 | wd |
| 4,2 | w |
| 3,94 | w |
| 3,82 | w |
| 3,7 | w |
| 3,3 | w |

w = faible
m = moyen
d = diffus

(forme β).

6. Procédé de préparation de la nouvelle forme cristalline stable du composé de formule I (forme β) selon la revendication 5, caractérisé en ce qu'on cristallise le composé de formule I dans une solution aqueuse à pH 3,5-7,5.

7. Composé de formule I selon la revendication 1 ou 5, pour l'utilisation comme produit intermédiaire pour la préparation de la ceftazidime.